# EUROPEAN PATENT APPLICATION

(11) **EP 4 846 947 A1**
(43) Date of publication of application: **02.12.2026**
(21) Application number: 25226811.5
(22) Date of filing: 23.12.2025
(51) Int. Cl.: A61B 17/04

(54) **SUTURING DEVICE**

(30) Priority: 29.05.2025 CN 202510717293
(71) Applicant: Micro-Tech (Nanjing) Co., Ltd., Nanjing, Jiangsu 210032 (CN); Zhang, Qiang, Guangzhou, Guangdong 510000 (CN)
(72) Inventor: ZHANG, Qiang, Guangzhou, Guangdong 510000 (CN); JIN, Hongyan, Nanjing, 210032 (CN); MA, Xiaojun, Nanjing, 210032 (CN); SONG, Ran, Nanjing, 210032 (CN); HU, Jie, Nanjing, 210032 (CN); LIN, Qi, Nanjing, 210032 (CN)
(74) Representative: Monteiro Alves, Inês

(57) **Abstract**

The present invention provides a suturing device, relating to the technical field of medical instruments. The suturing device provided in the present invention includes a suturing needle, a transmission member and a driving member. The suturing needle is in transmission connection with the transmission member, and the driving member is in transmission connection with the transmission member. The transmission member forms a channel corresponding to an endoscope. The suturing device is not only suitable for suturing operations in a narrow space of endoscopic surgeries, ensuring that the suturing needle can be driven smoothly, but also can avoid the obstruction of the suturing device on the field of view of the endoscope, ensuring the visibility of suturing operations under the endoscope, so that the suturing device is suitable for precise suturing operations in minimally invasive surgeries.

## Description

### TECHNICAL FIELD

The present invention relates to the technical field of medical devices, and in particular, to a suturing device.

### BACKGROUND ART

During endoscopic minimally invasive surgery, especially endoscopic minimally invasive surgery via the natural orifices of the digestive tract, a space at a surgical operation site is narrow. Therefore, the implementation of suturing operations faces significant challenges of space constraints. Due to the limitation of the surgical operation space, the layout of a transmission mechanism of a suturing device is strictly restricted. This restriction not only affects the mechanical performance of the device, leading to sluggish transmission and increased difficulty in suturing operations, but also can cause dual problems due to the obstruction of an endoscopic field of view by a control mechanism of the suturing device. First, an effective endoscopic field of view is significantly compressed. Second, it is difficult to obtain clear image information during a suturing operation, thereby affecting the accuracy and safety of the surgery. The superimposed effect of such space constraints and field-of-view interference places higher requirements on the precision of surgical operations. Next, some prior arts adopt the design of a C-shaped curved needle. Due to the use of a flat plate-like structure, the size of a suturing space at a C-shaped gap is small, which results in insufficient tissue capacity in the suturing space and limited puncture depth, thereby restricting the scope of application. Especially in flexible endoscopic minimally invasive surgery, it can only be used for simple wound suturing, and cannot be well applied to gastric volume reduction surgery involving gastrointestinal tissue folding.

### SUMMARY

The objective of the present invention is to provide a suturing device to solve the technical problem that a transmission mechanism of a suturing device obstructs a field of view of an endoscope and affects the visibility of suturing operations under the endoscope.

The suturing device provided in the present invention comprises a suturing needle, a transmission member, and a driving member. The suturing needle is in transmission connection with the transmission member, and the driving member is in transmission connection with the transmission member. The transmission member at least partially forms a channel corresponding to an endoscope.

Further, a rotation axis of the transmission member coincides with a rotation axis of the suturing needle.

Further, the channel extends axially from an inner side of the transmission member along a rotation axis of the transmission member and passes through an area enclosed by a movement trajectory of the suturing needle.

Further, the suturing needle is in intermittent and one-way transmission connection with the transmission member.

Further, a rotation axis of the transmission member is parallel to an axis of the endoscope, with a distance ranging from 0 to 5 mm.

Further, the driving member drives the transmission member through a rotational motion.

Further, the driving member comprises a flexible shaft and a driving gear. The driving gear is engaged with the transmission member for transmission, a distal end of the flexible shaft is connected to the driving gear, and the flexible shaft extends toward a proximal end along the endoscope.

Further, an outer ring or an inner ring of the transmission member is provided with a toothed portion adapted to the driving gear.

Further, the transmission member is rotatably installed inside a housing.

Further, the housing is provided with a side window facing the transmission member.

The side window comprises a wound tissue accommodating opening through which a movement trajectory of the suturing needle passes and a viewing opening facing the transmission member.

Further, the housing is provided with a sliding groove, and the suturing needle is in sliding fit with the sliding groove. The housing is movably connected to a needle cover, and the suturing needle is located between the housing and the needle cover.

Further, a support member is installed inside the housing, and the transmission member is installed between the support member and the housing.

Further, the support member is provided with an annular groove, and the transmission member is installed in the annular groove.

Further, the transmission member is provided with an elastic snapping portion, and the elastic snapping portion abuts against the suturing needle.

Further, the suturing needle is provided with a locking position adapted to the elastic snapping portion. In response to the transmission member rotating in a first direction, the elastic snapping portion engages with the locking position and drives a tip of the suturing needle to move in the first direction.

Further, the suturing needle is provided with an inclined surface extending from the locking position in a second direction opposite to the first direction. In response to the transmission member rotating in the second direction, the elastic snapping portion slides away from the locking position along the inclined surface.

Further, the suturing needle is provided with a one-way limiting portion. The suturing device further comprises an anti-retreat elastic plate adapted to the one-way limiting portion, and the anti-retreat elastic plate is inclined toward the suturing needle in the first direction. In response to the tip of the suturing needle moving in the first direction, the suturing needle slides relative to the anti-retreat elastic plate. In response to the transmission member rotating in a second direction opposite to the first direction, the anti-retreat elastic plate abuts against the one-way limiting portion to prevent the suturing needle from rotating in the second direction.

Further, the driving member comprises a first rack, a second rack, and a driving wheel assembly. The driving wheel assembly is in transmission connection with the transmission member. The first rack is spaced from the second rack, and the driving wheel assembly is engaged between the first rack and the second rack.

Further, the transmission member and the driving member are in transmission connection through a belt or a chain.

Further, the transmission member comprises an arc-shaped rack and a movable gear. The arc-shaped rack has a continuous toothed portion extending from an inner ring to an outer ring, and the movable gear meshes with the continuous toothed portion. Further, one of the arc-shaped rack and the movable gear is in transmission connection with the suturing needle, and another one of the arc-shaped rack and the movable gear is in transmission connection with the driving member.

Further, the transmission member is provided with an initial limiting portion and an end limiting portion that are arranged at an interval along a circumferential direction of the transmission member.

In response to the driving member driving the transmission member to reciprocate by switching a movement direction, the transmission member is located between an initial position where the initial limiting portion is engaged with the driving member and an end position where the end limiting portion is engaged with the driving member.

The embodiments of the present invention bring the following beneficial effects. By adopting the structure where the suturing needle is in transmission connection with the transmission member, the driving member is in transmission connection with the transmission member, and the transmission member at least partially forms the channel corresponding to the endoscope, not only the smooth driving of the suturing needle can be ensured, but also the obstruction of field of view of the endoscope by the suturing device can be avoid, thereby ensuring the visibility of suturing operations under the endoscope, so that the suturing device is suitable for precise suturing operations in minimally invasive surgeries. In addition, the channel formed by the enclosure of the transmission member can receive more tissue to enter the suturing action area of the suturing needle, thereby enabling the layered suturing of the wound site within the field of view of the endoscope, so that the suturing device has a wider application range.

To make the above objectives, features, and advantages of the present invention more clearly understandable, preferred embodiments are specifically cited below and described in detail with reference to drawings.

### BRIEF DESCRIPTION OF DRAWINGS

To more clearly illustrate the specific embodiments of the present invention or the technical solutions in the related art, the drawings that need to be used in the description of the specific embodiments or the related art will be briefly introduced below. Obviously, the drawings in the following description are some embodiments of the present invention, and those of ordinary skill in the art can also obtain other drawings based on these drawings without creative efforts.
FIG. 1 is a sectional view of one suturing device and an endoscope provided in an embodiment of the present invention;
FIG. 2 is a schematic diagram of one suturing device with a needle cover removed, provided in an embodiment of the present invention;
FIG. 3 is a schematic diagram of one suturing device provided in an embodiment of the present invention;
FIG. 4 is a schematic diagram of one suturing device with a housing and a needle cover removed, provided in an embodiment of the present invention;
FIG. 5 is an exploded view of another suturing device and endoscope provided in an embodiment of the present invention;
FIG. 6 is a sectional view of another suturing device and endoscope provided in an embodiment of the present invention;
FIG. 7 is an exploded view of another suturing device and endoscope provided in an embodiment of the present invention;
FIG. 8 is an exploded view of another suturing device and endoscope provided in an embodiment of the present invention;
FIG. 9 is an exploded view of another suturing device and endoscope provided in an embodiment of the present invention;
FIG. 10 is an exploded view of another suturing device and endoscope provided in an embodiment of the present invention;
FIG. 11 is a schematic diagram of a suturing needle provided in an embodiment of the present invention;
FIG. 12 is a schematic diagram of a transmission member for a suturing needle provided in an embodiment of the present invention;
FIG. 13 is a schematic diagram of a suturing device in a state where a needle cover is open, provided in an embodiment of the present invention;
FIG. 14 is a sectional view of a suturing device when a transmission member is at an initial position, provided in an embodiment of the present invention;
FIG. 15 is a sectional view of a suturing device when a transmission member is at an end position, provided in an embodiment of the present invention;
FIG. 16 is a schematic diagram of a first rack, a second rack, a driving wheel assembly, a transmission member, and a suturing needle of a suturing device provided in an embodiment of the present invention;
FIG. 17 is a schematic diagram showing a state of a suturing device during a suturing operation, provided in an embodiment of the present invention; and
FIG. 18 is a schematic diagram showing another state of a suturing device during a suturing operation, provided in an embodiment of the present invention.

Reference numerals: 100-suturing needle; 101-locking position; 102-inclined surface; 103-one-way limiting portion; 200-transmission member; 201-channel; 202-toothed portion; 203-elastic snapping portion; 204-initial limiting portion; 205-end limiting portion; 210-arc-shaped rack; 211-continuous toothed portion; 220-movable gear; 300-driving member; 310-flexible shaft; 320-driving gear; 330-first rack; 340-second rack; 350-driving wheel assembly; 351-cylindrical gear; 352-bevel gear; 400-housing; 401-side window; 402-sliding groove; 500-support member; 501-annular groove; 502- fixing ring; 600-anti-retreat elastic plate; 700-needle cover; 800-endoscope; 900-tissue to be sutured.

### DETAILED DESCRIPTION OF EMBODIMENTS

The technical solutions of the present invention will be clearly and completely described below with reference to the drawings. Obviously, the described embodiments are part of the embodiments of the present invention, rather than all the embodiments. Based on the embodiments of the present invention, all other embodiments obtained by those of ordinary skill in the art without creative efforts shall fall within the protection scope of the present invention.

In the description of the present invention, it should be noted that an orientation or positional relationship indicated by the term such as "center", "upper", "lower", "left", "right", "vertical", "horizontal", "inner" and "outer", is based on an orientation or positional relationship shown in the drawings, which is only for the convenience of describing the present invention and simplifying the description, rather than indicating or implying that the referred device or element must have a specific orientation, be constructed and operated in a specific orientation, and thus shall not be construed as a limitation to the present invention. In addition, the terms "first", "second", and "third" are only used to distinguish names and shall not be understood as indicating or implying relative importance. Physical quantities in a formula, unless individually specified, shall be understood as basic quantities of base units in the International System of Units, or derived quantities obtained from the basic quantities through mathematical operations such as multiplication, division, differentiation, or integration.

In the description of the present invention, it should be noted that unless otherwise clearly specified and limited, the terms such as "install", "link", and "connect" should be interpreted broadly. For example, the term "connection" can be a fixed connection, a detachable connection, or an integral connection; can be a mechanical connection, or an electrical connection; can be a direct connection, or an indirect connection through an intermediate medium; can be an internal communication between two components. For those of ordinary skill in the art, the specific meanings of the above terms in the present invention can be understood according to specific situations.

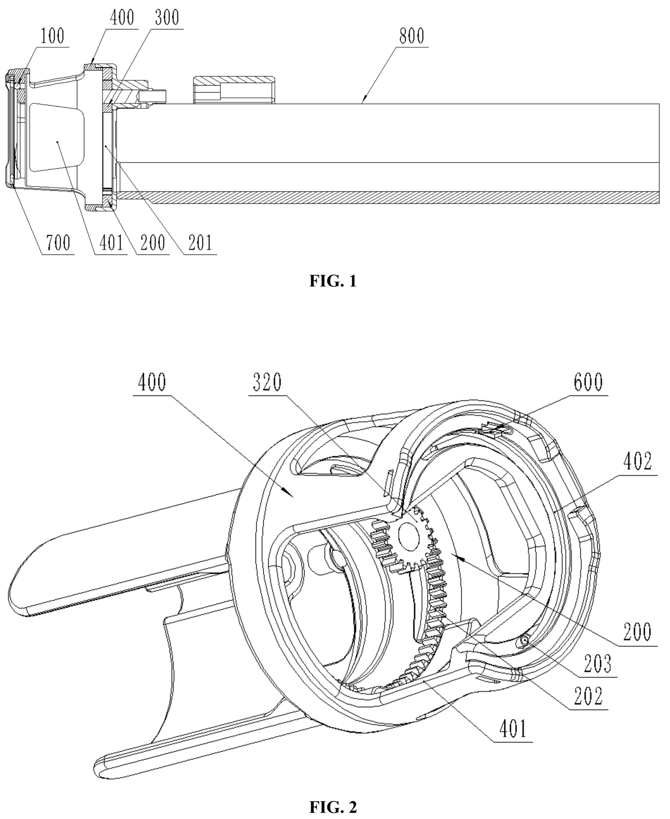

As shown in FIG. 1, a suturing device provided in an embodiment of the present invention includes a suturing needle 100, a transmission member 200, and a driving member 300. The suturing needle 100 is in transmission connection with the transmission member 200, and the driving member 300 is in transmission connection with the transmission member 200. The transmission member 200 at least partially forms a channel 201 corresponding to an endoscope 800. The transmission member 200 can be configured as an annular structure around the channel 201, or can extend into an arc-shaped structure along a circumferential direction of the channel 201.

In an optional embodiment, the driving member 300 can provide rotational power manually or electrically. The driving member 300 drives the transmission member 200, and the transmission member 200 drives the suturing needle 100 to move through means such as gear engagement transmission, belt transmission, or frictional contact. The channel 201 can reduce the obstruction of the suturing device to the field of view of the endoscope 800, thereby enabling suturing operations under a condition that the endoscope 800 provides clear images, which is conducive to improving the accuracy and safety of suturing operations in minimally invasive surgeries.

In an optional embodiment, a rotation axis of the transmission member 200 coincides with a rotation axis of the suturing needle 100. The channel 201 extends axially from an inner side of the transmission member 200 along the rotation axis of the transmission member 200 and passes through an area enclosed by a movement trajectory of the suturing needle 100. When the transmission member 200 is driven to rotate, the transmission member 200 drives the suturing needle 100 to move around the same rotation axis, which not only ensures smooth transmission, but also ensures that the endoscope 800 can pass through the channel 201 and the area enclosed by the movement trajectory of the suturing needle 100 in sequence to form a good field of view.

In an optional embodiment, the suturing needle 100 is in intermittent and one-way transmission connection with the transmission member 200. The transmission member 200 can drive the suturing needle 100 to perform suturing actions through one-way transmission components such as ratchets. After a suturing action is completed, the suturing needle 100 can continue to rotate along the movement trajectory, and thus move to an initial position of the suturing needle 100 again.

In an optional embodiment, the rotation axis of the transmission member 200 is parallel to an axis of the endoscope 800, with a distance ranging from 0 to 5 mm. In a case where the endoscope 800 is slightly offset relative to the rotation axis of the transmission member 200, under a condition of ensuring a sufficient field of view of the endoscope 800, an operation state of the transmission member 200 is able to be observed through the endoscope 800. In addition, the endoscope 800 can be offset relative to the rotation axis of the transmission member 200 toward an area of a suturing operation of the suturing needle 100, so as to obtain a better imaging effect on a wound suturing site.

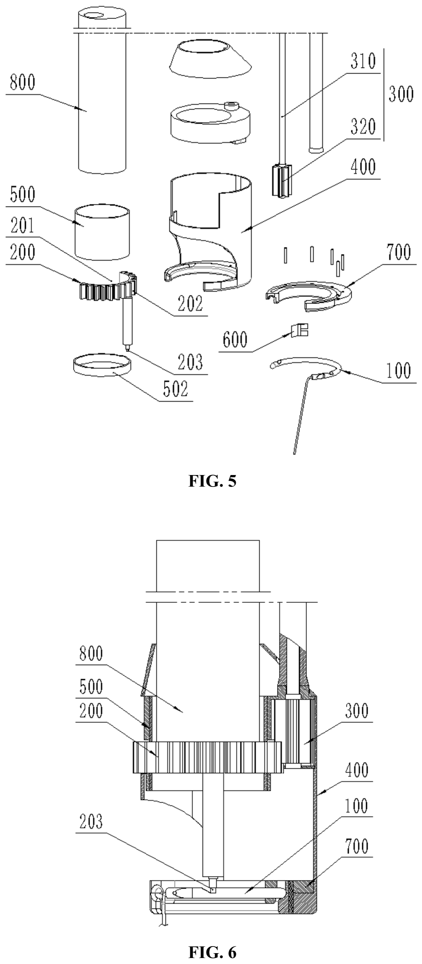

As shown in FIGS. 1 and 6, in an optional embodiment, the rotation axis of the transmission member 200 is parallel to and spaced from the axis of the endoscope 800, or the rotation axis of the transmission member 200 forms an included angle with the axis of the endoscope 800.

A distal end of the suturing device has a notch for the suturing needle 100 to pass through and move, and the endoscope 800 can be deflected toward this notch. During suturing, the movements of the suturing needle 100 can be clearly observed through the endoscope 800. After suturing, the suturing needle 100 moves away from the notch, and the field of view of the endoscope 800 extends outward from the notch, so that a wider field of view can be obtained.

As shown in FIGS. 1, 2, 3, 4, 5, 6, and 7, the driving member 300 drives the transmission member 200 through a rotational motion. A rigid or flexible shaft rod extending along the endoscope 800 to a proximal end (operation end) can be used. An operator can drive the transmission member 200 by twisting the shaft rod. The transmission mechanism needs a smaller layout space, so that the operation is more convenient.

Further, the driving member 300 includes a flexible shaft 310 and a driving gear 320. The driving gear 320 is engaged with the transmission member 200 for transmission. A distal end of the flexible shaft 310 is connected to the driving gear 320, and the flexible shaft 310 extends toward the proximal end along the endoscope 800.

The driving gear 320 can be configured as a cylindrical gear or a bevel gear. The flexible shaft 310 is connected to the driving gear 320, and is relatively and axially fixed. The driving gear 320 is in meshing transmission with the transmission member 200. By twisting the flexible shaft 310, the driving gear 320 can be driven to rotate, and the rotating driving gear 320 drives the transmission member 200 to rotate.

It should be noted that in an optional embodiment, a number of teeth of the driving gear 320 is less than a number of teeth of the transmission member 200. On one hand, an axial dimension of the transmission member 200 is larger, so as to form a larger channel 201, and on another hand, a driving force required by the driving gear 320 is smaller, making the twisting operation more labor-saving.

As shown in FIGS. 2, 3, 4, 5, 6, 7, 8, and 10, an outer ring, an inner ring or an axial end face of the transmission member 200 is provided with a toothed portion 202 adapted to the driving gear 320.

In an optional embodiment, the outer ring of the transmission member 200 is provided with the toothed portion 202, and the driving gear 320 is engaged with the outer ring of the transmission member 200. Thus, the inner ring of the transmission member 200 is entirely used to form the channel 201, which can ensure the field of view of the endoscope 800 to a maximum extent.

In another optional embodiment, the inner ring of the transmission member 200 is provided with the toothed portion 202, and the driving gear 320 is engaged with the inner ring of the transmission member 200. The driving gear 320 is installed inside the transmission member 200, and thus, a radial dimension of the suturing device can be reduced, so that the suturing device is especially suitable for minimally invasive surgeries with small wounds.

As shown in FIGS. 1 to 10, the transmission member 200 is rotatably installed inside a housing 400, and the housing 400 can provide an installation space for the suturing needle 100, the transmission member 200 and the driving member 300.

Further, the housing 400 is provided with a side window 401 facing the transmission member 200. The side window 401 includes a wound tissue accommodating opening through which the movement trajectory of the suturing needle 100 passes and a viewing opening facing the transmission member 200. The viewing opening can provide a wider field of view for the endoscope 800, and a working position and a state of the transmission member 200 can be observed through the viewing opening before and after the operation. During suturing, the tissue at the wound site can enter the wound tissue accommodating opening, and the suturing needle 100 can achieve the suturing action on the wound site by passing through the wound tissue accommodating opening.

As shown in FIGS. 1, 2, 3, and 4, the housing 400 is provided with a sliding groove 402, and the suturing needle 100 is in sliding fit with the sliding groove 402. The housing 400 is movably connected to a needle cover 700, and the suturing needle 100 is located between the housing 400 and the needle cover 700, which, on one hand, facilitates the assembly of the suturing needle 100; and on the other hand, the suturing needle 100 can be replaced by disassembling the needle cover 700 when the suturing needle 100 is damaged.

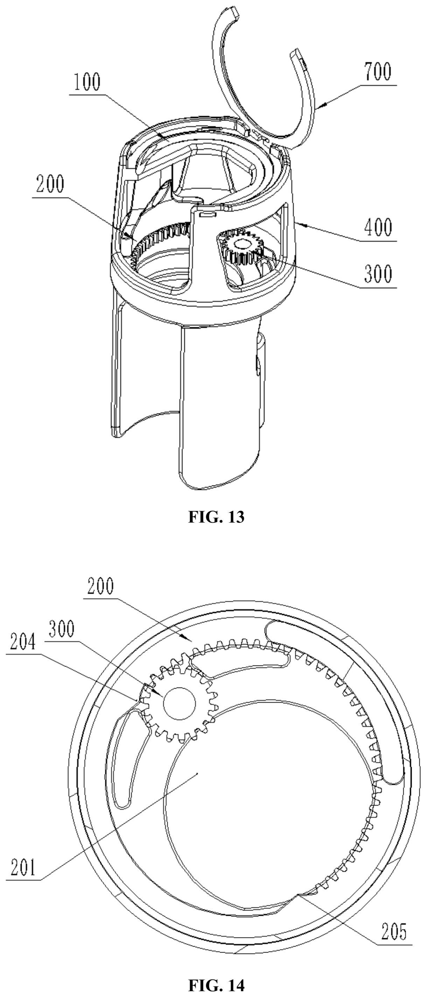

Referring to FIG. 13, the needle cover 700 can be opened and closed in a sliding or hinged manner relative to the housing 400. In a state where the needle cover 700 is open, the suturing needle 100 can be disassembled and cleaned.

Referring to FIG. 2, during a suturing movement, the suturing needle 100 slides along the sliding groove 402, and the movement trajectory of the suturing needle 100 is restricted by the sliding groove 402, which ensures the smooth and stable movement of the suturing needle 100.

In an optional embodiment, the housing 400 is provided with a stroke limiting portion corresponding to the transmission member 200, and the transmission member 200 rotates relative to the housing 400 within a limiting range of the stroke limiting portion. The stroke limiting portion can be configured as a limiting groove adapted to the transmission member 200, or as limiting protrusions arranged respectively at two ends of a rotation stroke of the transmission member 200, so as to restrict an angular range of the rotation stroke of the transmission member 200.

In addition, the limiting groove adapted to the transmission member 200 can be arranged on an inner side of the sliding groove 402, and the limiting groove extends in an arc shape around the rotation axis of the transmission member 200. The transmission member 200 is in transmission connection with the suturing needle 100 through an elastic snapping portion 203, and the elastic snapping portion 203 is in sliding fit within the limiting groove, thereby realizing the limitation on the rotation stroke of the transmission member 200.

As shown in FIGS. 5, 6, 7, 8, 9, and 10, a support member 500 is installed inside the housing 400, and the transmission member 200 is installed between the support member 500 and the housing 400.

The transmission member 200 and the support member 500 are arranged coaxially, and the support member 500 can support the transmission member 200 along a radial direction.

In an optional embodiment, the transmission member 200 can be configured as an annular structure or an arc-shaped structure extending along a circumferential direction of the support member 500. The transmission member 200 can slide smoothly around the support member 500 between the support member 500 and the housing 400.

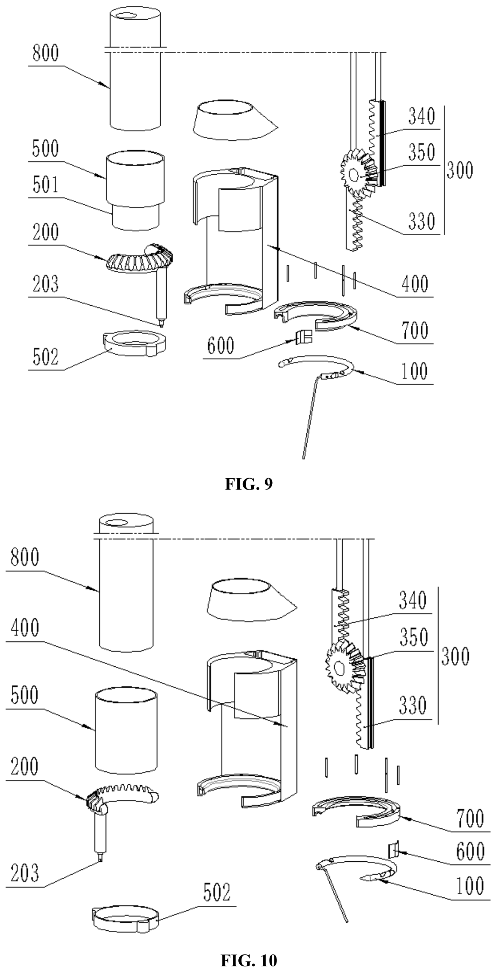

Referring to FIG. 9, the support member 500 is provided with an annular groove 501, and the transmission member 200 is installed inside the annular groove 501, which can reduce a radial dimension of the transmission member 200, thereby reducing an outer diameter of the suturing device, making the suturing device more suitable for surgeries with small wounds.

In addition, a fixing ring 502 is connected to the support member 500, and the fixing ring 502 abuts against an axial end of the transmission member 200, so that the transmission member 200 can be axially fixed relative to the support member 500.

As shown in FIGS. 2, 5, 6, 7, 8, 9, and 10, the transmission member 200 is provided with an elastic snapping portion 203, and the elastic snapping portion 203 abuts against the suturing needle 100. The elastic snapping portion 203 can be configured as a spring plunger or other elastic telescopic devices. By means of the elastic snapping portion 203 abutting against the suturing needle 100, when the transmission member 200 rotates, the elastic snapping portion 203 can drive the suturing needle 100 to move synchronously.

As shown in FIGS. 2, 5, 6, 7, 8, 9, 10, and 11, the suturing needle 100 is provided with a locking position 101 adapted to the elastic snapping portion 203. When the transmission member 200 rotates in a first direction, the elastic snapping portion 203 engages with the locking position 101 and drives a tip of the suturing needle 100 to move in the first direction, which can prevent the transmission member 200 from slipping relative to the suturing needle 100 during suturing driving.

In addition, the suturing needle 100 is provided with an inclined surface 102 extending from the locking position 101 in a second direction opposite to the first direction. When the transmission member 200 rotates in the second direction, the elastic snapping portion 203 detaches from the locking position 101 along the inclined surface 102. When the transmission member 200 is reversely driven to rotate in the second direction, the elastic snapping portion 203 slides away from the locking position 101 along the inclined surface 102, and the suturing needle 100 can remain stationary, and the transmission member 200 only drives the suturing needle 100 to perform suturing movements in the first direction.

Further, the suturing needle 100 is provided with a one-way limiting portion 103. The suturing device further includes an anti-retreat elastic plate 600 adapted to the one-way limiting portion 103, and the anti-retreat elastic plate 600 is inclined toward the suturing needle 100 in the first direction. When the tip of the suturing needle 100 moves in the first direction, the suturing needle 100 slides relative to the anti-retreat elastic plate 600. When the transmission member 200 rotates in the second direction opposite to the first direction, the anti-retreat elastic plate 600 abuts against the one-way limiting portion 103 to prevent the suturing needle 100 from rotating in the second direction, which improves the stability of the suturing needle 100 during one-way driving.

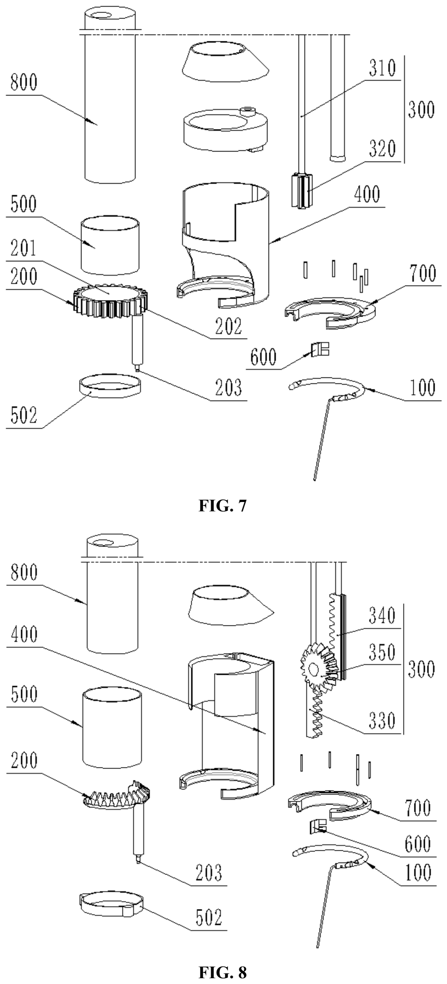

As shown in FIGS. 8, 9, and 10, the driving member 300 includes a first rack 330, a second rack 340, and a driving wheel assembly 350. The driving wheel assembly 350 is in transmission connection with the transmission member 200. The first rack 330 is spaced from the second rack 340, and the driving wheel assembly 350 is engaged between the first rack 330 and the second rack 340. Both the first rack 330 and the second rack 340 can be connected to a flexible shaft or a rod. One of the first rack 330 and the second rack 340 is subjected to a pushing force, and the other of the first rack 330 and the second rack 340 is subjected to a pulling force, so that the driving wheel assembly 350 can rotate. The driving wheel assembly 350 drives the transmission member 200, thereby realizing the suturing movement and the reset movement of the transmission member 200.

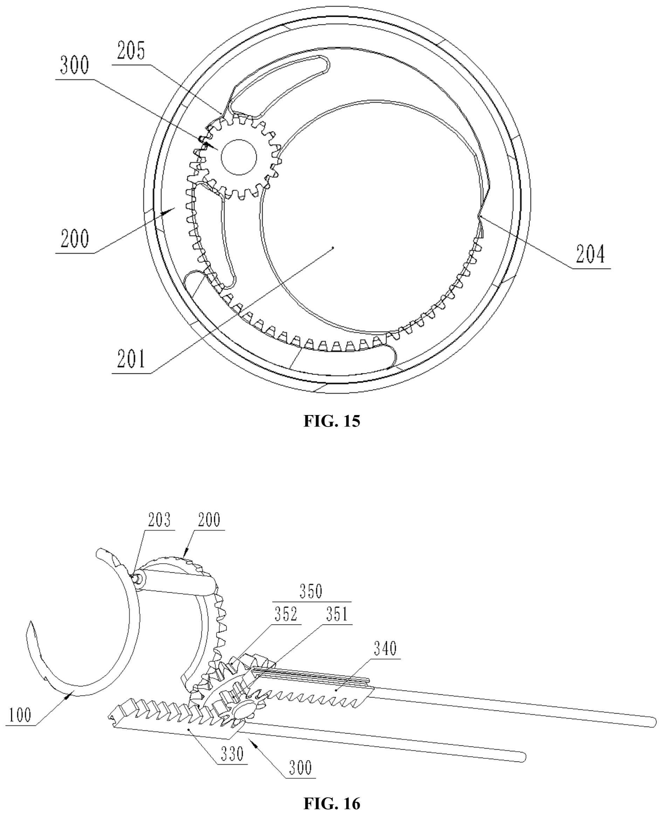

Referring to FIG. 16, the driving wheel assembly 350 can include a cylindrical gear 351 and a bevel gear 352 connected coaxially, and the cylindrical gear 351 and the bevel gear 352 can also be processed into an integrated structure. The cylindrical gear 351 is engaged between the first rack 330 and the second rack 340, and the bevel gear 352 is used to engage with the transmission member 200, thereby realizing the driving of the transmission member 200.

In addition to the above embodiments, the transmission member 200 and the driving member 300 can also be in transmission connection through a belt or a chain.

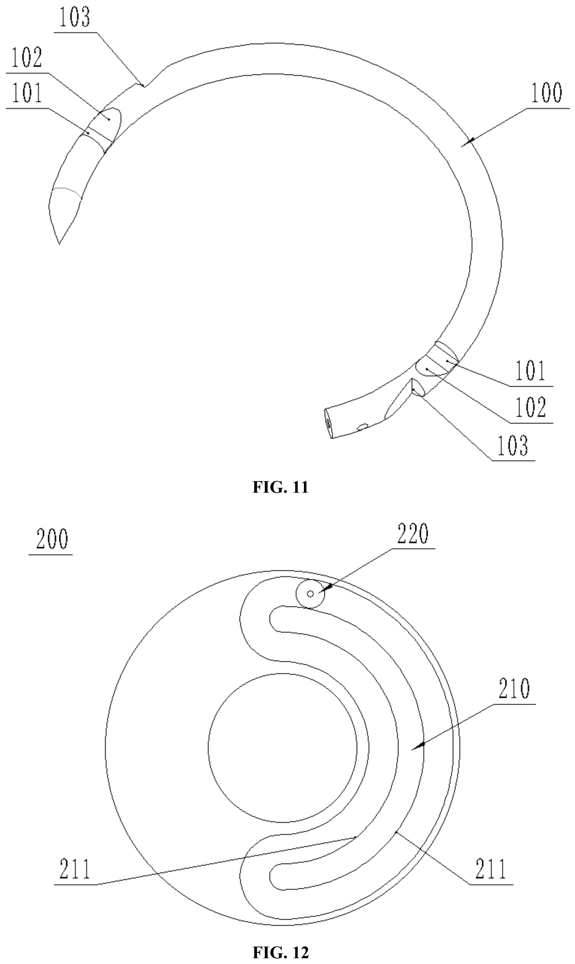

As shown in FIG. 12, in an optional embodiment, the transmission member 200 includes an arc-shaped rack 210 and a movable gear 220. The arc-shaped rack 210 has a continuous toothed portion 211 extending from an inner ring to an outer ring, and the movable gear 220 meshes with the continuous toothed portion 211. One of the arc-shaped rack 210 and the movable gear 220 is in transmission connection with the suturing needle 100, and another of the arc-shaped rack 210 and the movable gear 220 is in transmission connection with the driving member 300.

When the movable gear 220 is driven to rotate by a flexible shaft, the movable gear 220 can move continuously along the inner ring and outer ring of the arc-shaped rack 210, thereby generating an approximately arc-shaped reciprocating movement. The movable gear 220 with the approximately arc-shaped reciprocating movement can drive the suturing needle 100 to perform the suturing movements. In addition, when the driving member 300 drives the arc-shaped rack 210 to reciprocate along an arc-shaped track, an external force such as an elastic force can be used to make the movable gear 220 keep meshing with the continuous toothed portion 211, and make the movable gear 220 move continuously and circularly along the inner ring and outer ring of the arc-shaped rack 210 with the movement of the arc-shaped rack 210. In this way, the reciprocating movement can be realized through one-way driving, thereby realizing the continuous driving of the suturing needle 100.

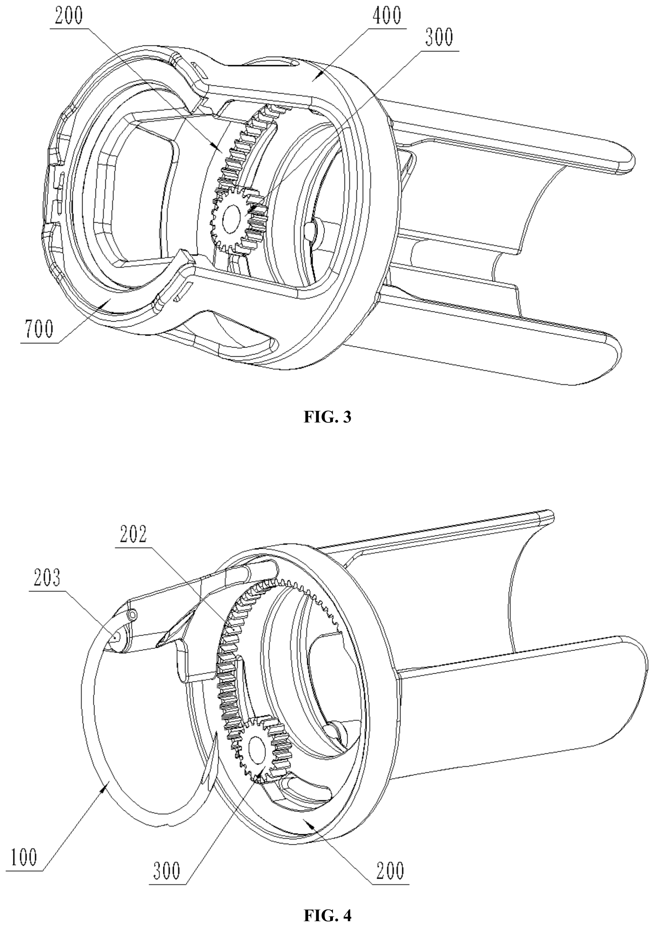

Referring to FIGS. 4, 14, and 15, in an optional embodiment, the transmission member 200 is provided with an initial limiting portion 204 and an end limiting portion 205 that are arranged at an interval along a circumferential direction of the transmission member 200. When the driving member 300 drives the transmission member 200 to reciprocate by switching the movement direction, the transmission member 200 is located between an initial position where the initial limiting portion 204 is engaged with the driving member 300 and an end position where the end limiting portion 205 is engaged with the driving member 300. When the driving member 300 drives the transmission member 200 until the end limiting portion 205 is engaged with the driving member 300, the end limiting portion 205 is blocked by the driving member 300, which can cause the driving member 300 to generate an obvious movement resistance. At this time, the operator can know that the transmission member 200 reaches a stroke limit position by touch, and then operate the driving member 300 in reverse until the transmission member 200 resets to the initial position where the initial limiting portion 204 is engaged with the driving member 300. By repeating this process, the driving and stroke limitation of the reciprocating rotation of the transmission member 200 can be realized.

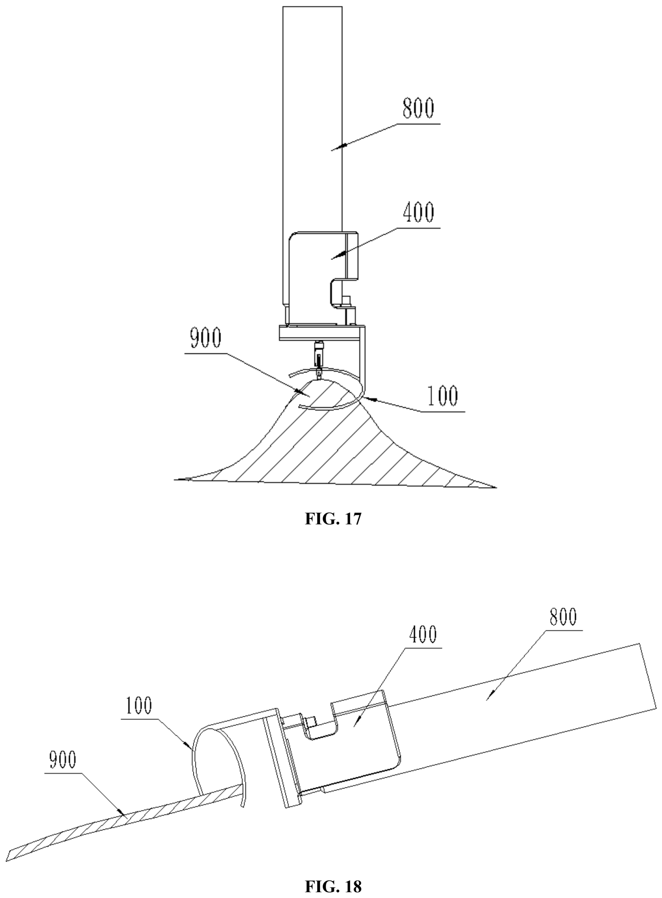

It should be emphasized that the present technology has requirements on the definition of spatial positions and functional adaptation relationships, which as a whole brings significantly different technical effects and solves various complex minimally invasive surgery problems. The design provides a simple and reliable structure, improves the operational space and field of view, ensures operation safety, and broadens the application scope. In addition to the above advantages of the structural features, an axial depth of the channel 201 can be adjusted according to the requirements of different endoscopic minimally invasive surgeries. For example, in order to receive more tissue to increase a folding height of gastrointestinal tissue, it is only necessary to lengthen the housing 400 axially, thereby pulling more tissue into the channel 201, and this does not change the design and stability of the structure. Referring to FIG. 17, medical forceps can be used to clamp a tissue 900 to be sutured and pull it into the operation space of the suturing needle 100, so as to perform the suturing operations by the suturing needle. The suturing method with the help of medical forceps accurately positions the tissue 900 to be sutured through the medical forceps, and the medical forceps can also be used to clamp and pull the tissue 900 to be sutured, thereby adjusting a depth of suturing or a height of gastrointestinal folding. Referring to FIG. 18, when the suturing device is advanced, the tissue 900 to be sutured can be inserted into the action space of the C-shaped opening of the suturing needle 100, and the suturing of the tissue 900 to be sutured can also be realized without additional use of medical forceps for auxiliary operation, resulting in higher suturing operation efficiency.

Finally, it should be noted that the above embodiments are only used to illustrate the technical solutions of the present invention, but not to limit them. Although the present invention is described in detail with reference to the foregoing embodiments, those of ordinary skill in the art should understand that they can still modify the technical solutions recorded in the foregoing embodiments, or make equivalent substitutions for some or all of the technical features, and these modifications or substitutions do not make the essence of the corresponding technical solutions deviate from the scope of the technical solutions of the embodiments of the present invention.

## Claims

1. A suturing device, **characterized by** comprising a suturing needle (100), a transmission member (200), and a driving member (300), wherein the suturing needle (100) is in transmission connection with the transmission member (200), and the driving member (300) is in transmission connection with the transmission member (200), wherein
the transmission member (200) at least partially forms a channel (201) corresponding to an endoscope (800).

2. The suturing device according to claim 1, wherein a rotation axis of the transmission member (200) coincides with a rotation axis of the suturing needle (100).

3. The suturing device according to claim 1, wherein the channel (201) extends axially from an inner side of the transmission member (200) along a rotation axis of the transmission member (200) and passes through an area enclosed by a movement trajectory of the suturing needle (100).

4. The suturing device according to claim 1, wherein the suturing needle (100) is in intermittent and one-way transmission connection with the transmission member (200).

5. The suturing device according to claim 1, wherein a rotation axis of the transmission member (200) is parallel to an axis of the endoscope (800), with a distance ranging from 0 to 5 mm.

6. The suturing device according to claim 1, wherein the driving member (300) drives the transmission member (200) through a rotational motion.

7. The suturing device according to claim 6, wherein the driving member (300) comprises a flexible shaft (310) and a driving gear (320), wherein
the driving gear (320) is engaged with the transmission member (200) for transmission, a distal end of the flexible shaft (310) is connected to the driving gear (320), and the flexible shaft (310) extends toward a proximal end along the endoscope (800).

8. The suturing device according to claim 1, wherein the transmission member (200) is rotatably installed inside a housing (400).

9. The suturing device according to claim 8, wherein the housing (400) is provided with a side window (401), wherein
the side window (401) comprises a wound tissue accommodating opening through which a movement trajectory of the suturing needle (100) passes and a viewing opening facing the transmission member (200).

10. The suturing device according to claim 8, wherein the housing (400) is provided with a sliding groove (402), and the suturing needle (100) is in sliding fit with the sliding groove (402); and
the housing (400) is movably connected to a needle cover (700), and the suturing needle (100) is located between the housing (400) and the needle cover (700).

11. The suturing device according to claim 8, 9 or 10, wherein the housing (400) is provided with a stroke limiting portion corresponding to the transmission member (200), and the transmission member (200) rotates relative to the housing (400) within a limiting range of the stroke limiting portion.

12. The suturing device according to claim 8, wherein a support member (500) is installed inside the housing (400), and the transmission member (200) is installed between the support member (500) and the housing (400).

13. The suturing device according to claim 12, wherein the support member (500) is provided with an annular groove (501), and the transmission member (200) is installed in the annular groove (501).

14. The suturing device according to claim 1, wherein the transmission member (200) is provided with an elastic snapping portion (203), and the elastic snapping portion (203) abuts against the suturing needle (100), wherein
the suturing needle (100) is provided with a locking position (101) adapted to the elastic snapping portion (203), wherein
in response to the transmission member (200) rotating in a first direction, the elastic snapping portion (203) engages with the locking position (101) and drives a tip of the suturing needle (100) to move in the first direction;
the suturing needle (100) is provided with an inclined surface (102) extending from the locking position (101) in a second direction opposite to the first direction, wherein
in response to the transmission member (200) rotating in the second direction, the elastic snapping portion (203) slides away from the locking position (101) along the inclined surface (102), wherein
the suturing needle (100) is provided with a one-way limiting portion (103); and
the suturing device further comprises an anti-retreat elastic plate (600) adapted to the one-way limiting portion (103), and the anti-retreat elastic plate (600) is inclined toward the suturing needle (100) in the first direction, wherein
in response to the tip of the suturing needle (100) moving in the first direction, the suturing needle (100) slides relative to the anti-retreat elastic plate (600);
in response to the transmission member (200) rotating in a second direction opposite to the first direction, the anti-retreat elastic plate (600) abuts against the one-way limiting portion (103) to prevent the suturing needle (100) from rotating in the second direction.

15. The suturing device according to claim 1, wherein the driving member (300) comprises a first rack (330), a second rack (340), and a driving wheel assembly (350), wherein
the driving wheel assembly (350) is in transmission connection with the transmission member (200); and
the first rack (330) is spaced from the second rack (340), and the driving wheel assembly (350) is engaged between the first rack (330) and the second rack (340);
the transmission member (200) comprises an arc-shaped rack (210) and a movable gear (220), wherein
the arc-shaped rack (210) has a continuous toothed portion (211) extending from an inner ring to an outer ring, and the movable gear (220) meshes with the continuous toothed portion (211); and
one of the arc-shaped rack (210) and the movable gear (220) is in transmission connection with the suturing needle (100), and another one of the arc-shaped rack (210) and the movable gear (220) is in transmission connection with the driving member (300); and the transmission member (200) is provided with an initial limiting portion (204) and an end limiting portion (205) that are arranged at an interval along a circumferential direction of the transmission member (200), wherein
in response to the driving member (300) driving the transmission member (200) to reciprocate by switching a movement direction, the transmission member (200) is located between an initial position where the initial limiting portion (204) is engaged with the driving member (300) and an end position where the end limiting portion (205) is engaged with the driving member (300).
